# EUROPEAN PATENT SPECIFICATION

(11) **EP 2 661 244 B1**
(45) Date of publication and mention of the grant of the patent: **07.10.2015**
(21) Application number: 12700889.4
(22) Date of filing: 05.01.2012
(51) Int. Cl.: A61F 2/97, A61F 2/962

(54) **PROXIMAL RELEASE EXPANDABLE PROSTHESIS DELIVERY SYSTEM**
SYSTEM ZUR VERABREICHUNG DEHNBARER PROTHESEN MIT PROXIMALER FREISETZUNG
SYSTÈME DE POSE À LIBÉRATION PROXIMALE POUR PROTHÈSE EXPANSIBLE

(30) Priority: 05.01.2011 US 984893
(43) Date of publication of application: 13.11.2013
(73) Proprietor: Cook Medical Technologies LLC, Bloomington, IN 47404 (US)
(72) Inventor: HATFIELD, Adrian, Surrey KT6 6BU (GB)
(74) Representative: Garratt, Peter Douglas
(86) International application number: PCT/US2012/020277
(87) International publication number: WO 2012/094458

(56) References cited:
- EP-A2- 1 985 261
- WO-A1-2009/050265
- WO-A2-03/039345
- US-A1- 2006 089 627
- US-A1- 2008 167 705
- US-A1- 2009 204 196

## Description

### BACKGROUND

The present invention relates generally to medical devices and more specifically to delivery systems for expandable prostheses.

Use of expandable prostheses such as self-expanding stents is well known in the art. Most of the known delivery systems for such prostheses deploy the prosthesis in the distal to proximal direction, where "proximal" and "distal" are defined relative to the operator. However, it is advantageous to deploy a prosthesis in the proximal to distal direction when the placement of the proximal portion of the prosthesis is critical.

For example, use of self-expanding stents within the gastrointestinal (GI) tract is well-known. However, foreshortening stents tend to anchor within the GI tract at the initial location the stent makes contact with the body vessel, and then shorten along their primary axis toward that location. Thus, when using a foreshortening stent, the end that initially opens is relatively easy to place with accuracy, but the final location of the opposite end of the stent is variable.

There may be a clinical need to achieve precise anatomical placement of the proximal end of the self-expanding stent. For example, when there is a need to place a self-expanding stent very high in the esophagus, the proximal end of the stent should ideally be above the stricture but below the cricopharyngeal region of the throat to avoid aggravation of the nerves that control the coughing response and to avoid blocking the cricopharyngeal junction.

Thus, there is a need for a simple and reliable device to accurately position the proximal portion of an expandable prosthesis in the prosthesis's deployed configuration.

The use of splittable sheaths, such Cook Medical's as Peel-Away® sheath technology, to facilitate the insertion of catheters into body vessels is also known in the art. For example, US Pat. No. 4,306.562 to Osborne describes a cannula that is configured to tear into two pieces as it is withdrawn from the body vessel in order to accommodate fittings, connectors, or other items larger than the cannula at the proximal end of the device. WO 03/039345 discloses a delivery device having a guide member with a plurality of struts disposed at the distal end. A sleeve surrounds the struts and applies a force against the struts to prevent the struts from extending outwardly or to maintain a dilation balloon or stent within the lumen. Disposed within the sleeve or between the sleeve and the guide member are one or more actuating members which extend to the distal end of the sleeve and subsequently extend proximally on the outside of the sleeve to terminate at the proximal end of the device. Displacing the actuating members 228 in the proximal direction causes the actuating members to preferentially separate the sleeve into one or more portions, so releasing the struts. Reference is also directed to WO 2009/050265, US 2009/204196, US 2008/167705 and US 2006/089627 which disclose various delivery devices having splittable sheaths with some splitters including blade elements.

### BRIEF SUMMARY

The invention may include any of the following aspects in various combinations and may also include any other aspect described below in the written description or in the attached drawings, as for as covered by the appended claims.

A medical device includes an elongate catheter with a proximal end, a distal end, and a lumen. An expandable prosthesis is disposed about the distal end of the elongate catheter. A splittable sheath with a proximal end and a distal end is disposed about the expandable prosthesis. The splittable sheath has an opening disposed upon its proximal end. Also included is at least one actuating wire with a first portion, a second portion, and a first end. The first end of the actuating wire is disposed on the first portion of the actuating wire. The first portion of the actuating wire is everted about the distal end of the elongate catheter and disposed between the expandable prosthesis and the splittable sheath. The second portion of the actuating wire is disposed within the lumen of the elongate catheter. Additionally, a splitter with a blade element is attached to the first end of the actuating wire and partially disposed within the opening on the proximal end of the splittable sheath. When an operator at the proximal end of the elongate catheter pulls the actuating wire in the proximal direction the splitter moves in the distal direction and the blade element splits the splittable sheath.

The blade element may be hook-shaped or knife-shaped.

The blade element may also include a protective guard that covers the splitter.

The splittable sheath may include a longitudinal weakness that extends distally from the opening in the proximal end of the splittable sheath.

The proximal release delivery system may also include an outer catheter disposed about the splittable sheath. The outer catheter is configured to be displaced in the proximal direction before the splittable sheath is split.

The splittable sheath may be composed of polytetrafluoroethylene (PTFE) or polyethylene (PE).

The proximal release delivery system may also include an opening through the wall of the elongate catheter disposed on the distal end of the elongate catheter. The first portion of the actuating wire passes through the opening through the wall of the elongate catheter on the distal end of the elongate catheter. This allows the distal end of the splittable sheath to be attached to the distal end of the elongate catheter distal of the opening on the distal end of the elongate catheter without interfering with the placement of the actuating wire.

Another embodiment of the medical device includes an elongate catheter with a proximal end, a distal end, and a lumen. An expandable prosthesis is disposed about the distal end of the elongate catheter. A hinged splittable sheath with a longitudinal hinge attached to the expandable prosthesis is disposed about the expandable prosthesis. An opening is disposed on the proximal end of the hinged splittable sheath. Also included is an actuating wire with a first portion, a second portion, and a first end. The first end of the actuating wire is disposed on the first portion of the actuating wire. The first portion of the actuating wire is everted about the distal end of the elongate catheter and disposed between the expandable prosthesis and the hinged splittable sheath. The second portion of the actuating wire is disposed within the lumen of the elongate catheter. A splitter with a blade element is attached to the first end of the actuating wire and partially disposed within the opening on the proximal end of the hinged splittable sheath. When an operator at the proximal end of the elongate catheter pulls the actuating wire in the proximal direction, the splitter moves in the distal direction and the blade element splits the hinged splittable sheath.

The hinged splittable sheath may be composed of polytetrafluoroethylene (PTFE) or polyethylene (PE).

### BRIEF DESCRIPTION OF THE DRAWINGS

FIG. 1 is a partial longitudinal view of the proximal release delivery system.
FIG. 2 is a partial longitudinal section view of the proximal release delivery system wherein the splittable sheath is attached to the distal end of the elongate catheter and the device includes an outer catheter.
FIG. 3 is a longitudinal view of the splittable sheath wherein the sheath includes a longitudinal weakness.
FIG. 4(a) is a longitudinal view of the splitter wherein the splitter is hook-shaped.
FIG. 4(b) is a longitudinal view of the splitter wherein the splitter is shaped like a knife.
FIG. 4(c) is a longitudinal view of the splitter including a protective guard.
FIG. 5 is a broken perspective view of the proximal release delivery system wherein the splittable sheath includes a longitudinal hinge.
FIG. 6 is a partial longitudinal section view of the proximal release delivery system wherein the splittable sheath includes a longitudinal hinge and the device includes an outer catheter.

### DETAILED DESCRIPTION OF THE DRAWINGS AND THE PRESENTLY PREFERRED EMBODIMENTS

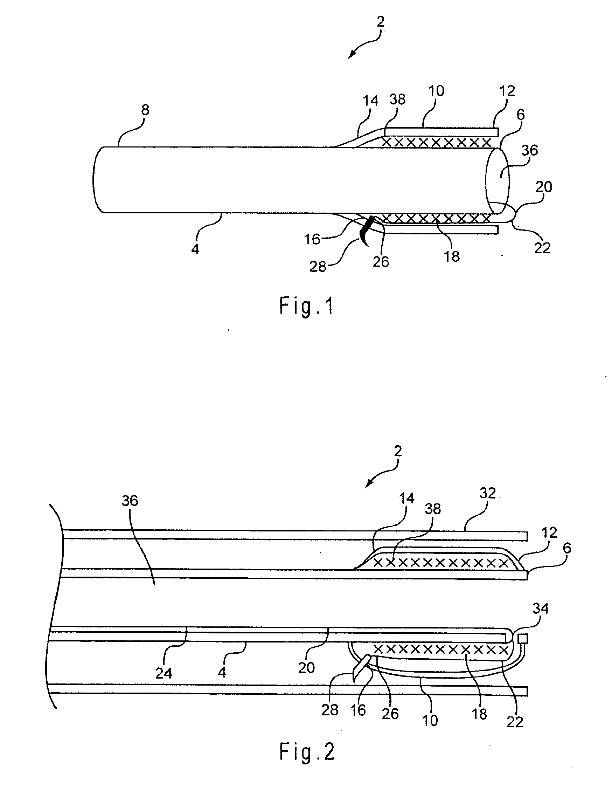

Referring now to the figures, a proximal release delivery system for expandable prostheses is shown. One embodiment of the delivery system is shown in FIG. 1. The proximal release delivery system 2 includes elongate catheter 4. Elongate catheter 4 has a distal end 6 and a proximal end 8. An expandable prosthesis 18 is disposed around the distal end 6 of elongate catheter 4.

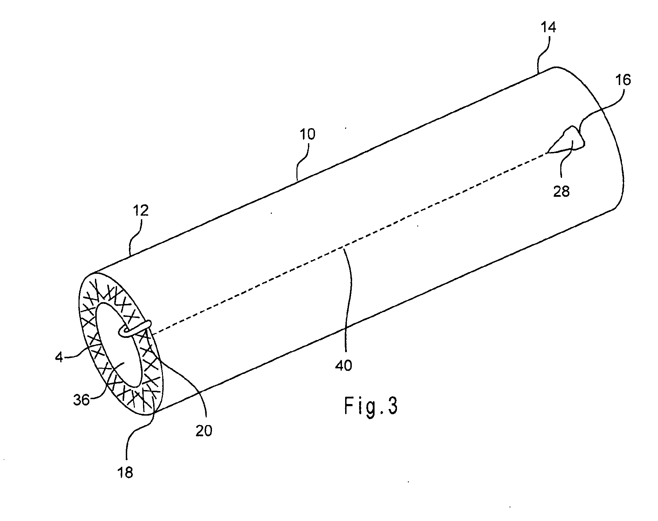

A splittable sheath 10 is disposed around expandable prosthesis 18 such that splittable sheath 10 retains expandable prosthesis 18 in a compressed configuration around elongate catheter 4. The splittable sheath 10 has a distal end 12 and a proximal end 14, where the proximal end 14 of splittable sheath 10 begins at the proximal end of expandable prosthesis 18. An opening 16 is located on the proximal end 14 of splittable sheath 10. Splittable sheath 10 may be manufactured of any of a number of well-known materials, including polytetrafluoroethylene (PTFE) and polyethylene (PE). Splittable sheath 10 may also be manufactured in such a way that it is predisposed to split longitudinally. For example, as shown in FIG. 3, a longitudinal weakness 40 could be pre-ground into the splittable sheath 10 to encourage the sheath to split longitudinally while still retaining adequate transverse strength to retain the expandable prosthesis 18. The longitudinal weakness 40 extends in the distal direction from opening 16 on the proximal end 14 of splittable sheath 10. Alternatively, the longitudinal weakness 40 could be formed by perforating the splittable sheath longitudinally from opening 16 on the proximal end 14 of splittable sheath 10 in the distal direction. Splittable sheath 10 may extend the full length of the delivery system; i.e. from the distal end 6 of elongate catheter 4 to an operator located at the proximal end 8 of elongate catheter 4.

An actuating wire 20 is partially disposed within the lumen 36 of elongate catheter 4. The actuating wire 20 has a first portion 22, a second portion 24, and a first end 26. The first end 26 is located on the end of the actuating wire 20 in the first portion 22. The first portion 22 of actuating wire 20 is everted about the distal end 6 of elongate catheter 4. The first portion 22 of actuating wire 20 then travels between expandable prosthesis 18 and splittable sheath 10 to the opening 16 located on the proximal end of splittable sheath 10. A splitter 28 having a blade element 42 is attached to the first end 26 of actuating wire 20 and is partially disposed within opening 16. The second portion 24 of actuating wire 20 travels within lumen 36 of elongate catheter 4 to the proximal end 8 of elongate catheter 4. The proximal release delivery system 2 may also include more than one actuating wire 20, splitter 28, and opening 16.

In order to deploy the expandable prosthesis 18, an operator located at the proximal end 8 of elongate catheter 4 advances proximal release delivery system 2 through a body vessel to the desired treatment location. Once the proximal end 38 of expandable prosthesis 18 is at the desired location, the operator pulls the second portion 24 of actuating wire 20 in the proximal direction causing the splitter 28 to move in the distal direction. As splitter 28 moves, blade element 42 splits the splittable sheath 10 longitudinally, releasing expandable prosthesis 18 and allowing it to deploy from the proximal end of the prosthesis. Thus, the proximal end 38 of expandable prosthesis 18 is accurately deployed at the desired location.

As shown in FIG. 2, an outer catheter 32 may be disposed around splittable sheath 10 to protect the splittable sheath 10 and the body vessel during placement of proximal release delivery system 2. The operator pulls outer catheter 32 in the proximal direction to fully uncover splittable sheath 10 before releasing the expandable prosthesis 18 as described above.

As shown in FIG. 2, the distal end 12 of splittable sheath 10 may be attached to the distal end 6 of elongate catheter 4 to facilitate removal of splittable sheath 10 after deployment. In order to allow actuating wire 20 to travel between the expandable prosthesis 18 and the splittable sheath 10 to the splitter 28 disposed in opening 16, an opening 34 through the wall of the elongate catheter 18 is disposed on the distal end 6 of elongate catheter 4. The first portion 22 of actuating wire 20 is threaded from lumen 36 through opening 34 and between expandable prosthesis 18 and the splittable sheath 10. After deployment, the operator withdraws elongate catheter 4 in the proximal direction to remove the proximal release delivery system 2 from the body lumen. If the distal end 12 of splittable sheath 10 is attached to the distal end 6 of elongate catheter 4, the splittable sheath 10 is removed along with elongate catheter 4.

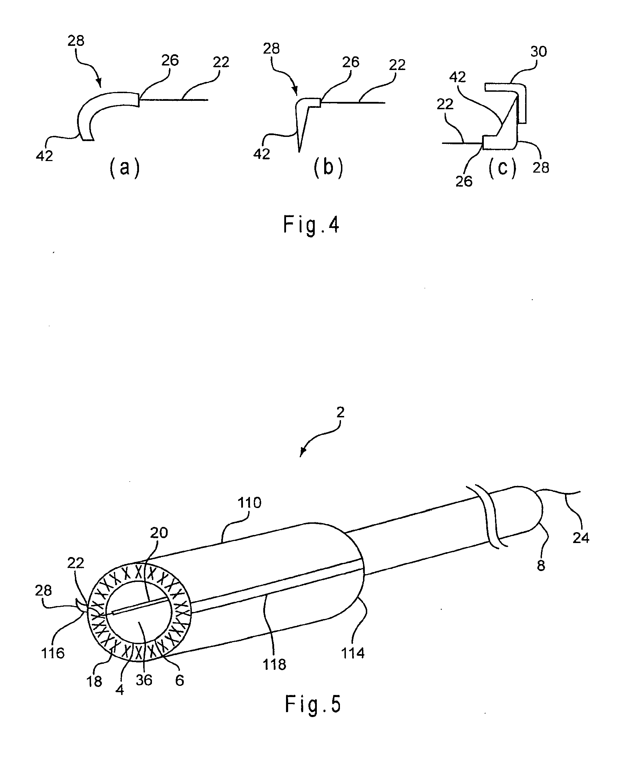

The blade element 42 of splitter 28 may be any shape effective for splitting splittable sheath 10. For example, as shown in FIG. 4(a), blade element 42 may be hook-shaped. FIG. 4(b) illustrates a knife-shaped blade element 42. And FIG. 4(c) shows a splitter 28 and blade element 42 covered by a protective guard 30. Protective guard 30 protects the body lumen and other medical devices from being damaged by blade element 42.

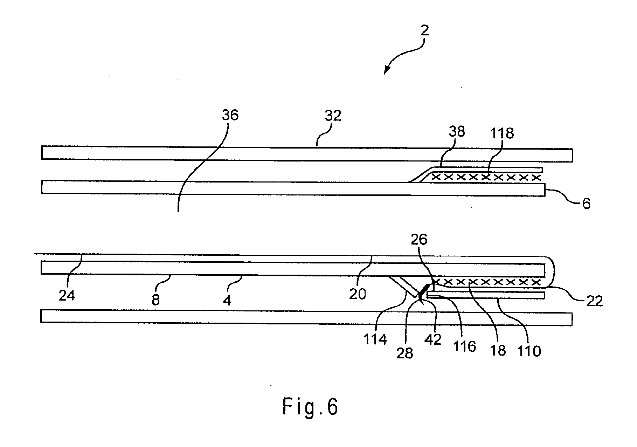

Another embodiment of proximal release delivery system 2 is shown in FIGs. 5 and 6. As shown in FIG. 5, a longitudinal hinge 118 runs along the entire length of hinged splittable sheath 110 in this embodiment. The longitudinal hinge 118 of hinged splittable sheath 110 may be created using one of many different techniques. These techniques include manufacturing a U or V shaped longitudinal groove in the tubing to create the hinged splittable sheath 110. Longitudinal hinge 118 is attached to the expandable prosthesis 18. Hinged splittable sheath 110 has a proximal end 114 and an opening 116 disposed on proximal end 114. Opening 116 is not disposed upon the longitudinal hinge 118. Actuating wire 20 is threaded between expandable prosthesis 18 and hinged splittable sheath 110 in the same fashion as shown in FIG. 1. Splitter 28 is attached to the first end 26 of actuating wire 20 and partially disposed within opening 116.

As the operator pulls the second portion 24 of actuating wire 20 in the proximal direction, splitter 28 with blade element 42 moves in the distal direction and splits hinged splittable sheath 110 longitudinally. As hinged splittable sheath 110 begins to split, the expandable prosthesis 18 expands and deploys. As it deploys, expandable prosthesis 18 forces hinged splittable sheath 110 to its fully opened position. Additionally, expandable prosthesis 18 traps the split and fully opened hinged splittable sheath 110 between the expandable prosthesis 18 and a body vessel wall. Because hinged splittable sheath 110 is attached to expandable prosthesis 18, hinged splittable sheath remains at the deployment location with expandable prosthesis 18 after the operator removes the rest of proximal release delivery system 2.

Hinged splittable sheath 110 does not interfere with the proper placement and deployment of expandable prosthesis 18 because the surface area of hinged splittable sheath 110 is significantly smaller than outer surface area of expandable prosthesis 18 in its expanded configuration. As shown in FIG. 6, this embodiment may also include an outer catheter 32 to protect hinged splittable sheath 110 and the body vessel during deployment to the treatment site.

Hinged splittable sheath 110 may also extend along the full length of proximal release delivery system 2. The proximal release delivery system 2 of this embodiment may also include more than one splitter 28.

While preferred embodiments of the invention have been described, it should be understood that the invention is not so limited, and modifications may be made without departing from the invention. The scope of the invention is defined by the appended claims.

## Claims

1. A medical device comprising:
an elongate catheter (4) with a proximal end and distal end and a lumen;
an expandable prosthesis (18) disposed about said distal end of said elongate catheter;
a splittable sheath (10, 110) with a proximal end and a distal end disposed about said expandable prosthesis; said splittable sheath having an opening (16) disposed upon said proximal end of said splittable sheath;
at least one actuating wire (20) with a first portion (22), a second portion (24), and a first end (26); said first end disposed on said first portion of said actuating wire; said first portion of said actuating wire everted about said distal end of said elongate catheter and disposed between said expandable prosthesis and said splittable sheath; said second portion of said actuating wire disposed within said lumen of said elongate catheter;
**characterised by** a splitter (28) attached to said first end of said at least one actuating wire; said splitter including a blade element (42); said splitter partially disposed within said opening on said proximal end of said splittable sheath such that when an operator at said proximal end of said elongate catheter pulls said actuating wire in the proximal direction said splitter moves in the distal direction and said splitter blade element splits the splittable sheath.

2. The medical device of Claim 1 wherein said splitter blade element is hook-shaped.

3. The medical device of Claim 1 wherein said splitter blade element is knife-shaped.

4. The medical device of Claim 1 wherein a protective guard covers said splitter.

5. The medical device of Claim 1 wherein an outer catheter is disposed about said splittable sheath; said outer catheter configured to be displaced in the proximal direction before said splittable sheath is split.

6. The medical device of Claim 1 wherein said splittable sheath is composed of polytetrafluoroethylene (PTFE) or polyethylene (PE).

7. The medical device of Claim 1 wherein a longitudinal weakness is disposed upon said splittable sheath; said longitudinal weakness extending distally from said opening on said proximal end of said splittable sheath.

8. The medical device of Claim 1 wherein an opening is disposed on said distal end of said elongate catheter; said distal end of said splittable sheath is attached to said distal end of said elongate catheter distal of said opening on said distal end of said elongate catheter; and said first portion of said actuating wire passes through said opening on said distal end of said elongate catheter.

9. The medical device of Claim 1 wherein said medical device has a plurality of said actuating wires, a plurality of said openings on said proximal end of said splittable sheath, and a plurality of splitters.

10. The medical device of any one of Claim 1 to Claim 7, wherein said splittable sheath comprises a hinged splittable sheath having a longitudinal hinge attached to said expandable prosthesis.

11. The medical device of Claim 10 wherein said medical device has a plurality of said actuating wires, a plurality of said openings on said proximal end of said hinged splittable sheath, and a plurality of splitters.

## Patentansprüche

1. Medizinische Vorrichtung, umfassend:
einen länglichen Katheter (4) mit einem proximalen Ende und einem distalen Ende und einem Lumen,
eine expandierbare Prothese (18), die um das distale Ende des länglichen Katheters herum angeordnet ist,
eine spaltbare Hülse (10, 110) mit einem proximalen Ende und einem distalen Ende, die um die expandierbare Prothese herum angeordnet ist, wobei die spaltbare Hülse eine Öffnung (16) hat, die an dem proximalen Ende der spaltbaren Hülse angeordnet ist,
mindestens einen Betätigungsdraht (20) mit einem ersten Abschnitt (22), einem zweiten Abschnitt (24) und einem ersten Ende (26), wobei das erste Ende am ersten Abschnitt des Betätigungsdrahts angeordnet ist, wobei der erste Abschnitt des Betätigungsdrahts um das distale Ende des länglichen Katheters umgestülpt und zwischen der expandierbaren Prothese und der spaltbaren Hülse angeordnet ist, wobei der zweite Abschnitt des Betätigungsdrahts in dem Lumen des länglichen Katheters angeordnet ist,
**gekennzeichnet durch** einen Spalter (28), der am ersten Ende des mindestens einen Betätigungsdrahts angebracht ist, wobei der Spalter ein Messerelement (42) aufweist und teilweise in der Öffnung am proximalen Ende der spaltbaren Hülse angeordnet ist, so dass sich, wenn ein Bediener am proximalen Ende des länglichen Katheters in der proximalen Richtung an dem Betätigungsdraht zieht, der Spalter in die distale Richtung bewegt und das Spaltermesserelement die spaltbare Hülse spaltet.

2. Medizinische Vorrichtung nach Anspruch 1, wobei das Spaltermesserelement hakenförmig ist.

3. Medizinische Vorrichtung nach Anspruch 1, wobei das Spaltermesserelement messerförmig ist.

4. Medizinische Vorrichtung nach Anspruch 1, wobei eine Schutzvorrichtung den Spalter abdeckt.

5. Medizinische Vorrichtung nach Anspruch 1, wobei ein äußerer Katheter um die spaltbare Hülse herum angeordnet ist, wobei der äußere Katheter dazu konfiguriert ist, vor dem Spalten der spaltbaren Hülse in die proximale Richtung verschoben zu werden.

6. Medizinische Vorrichtung nach Anspruch 1, wobei die spaltbare Hülse aus Polytetrafluorethylen (PTFE) oder Polyethylen (PE) besteht.

7. Medizinische Vorrichtung nach Anspruch 1, wobei eine Längsschwachstelle an der spaltbaren Hülse angeordnet ist, wobei sich die Längsschwachstelle distal von der Öffnung am proximalen Ende der spaltbaren Hülse erstreckt.

8. Medizinische Vorrichtung nach Anspruch 1, wobei eine Öffnung am distalen Ende des länglichen Katheters angeordnet ist, das distale Ende der spaltbaren Hülse distal von der Öffnung am distalen Ende des länglichen Katheters am distalen Ende des länglichen Katheters angebracht ist und der erste Abschnitt des Betätigungsdrahts durch die Öffnung am distalen Ende des länglichen Katheters geht.

9. Medizinische Vorrichtung nach Anspruch 1, wobei die medizinische Vorrichtung mehrere der Betätigungsdrähte, mehrere der Öffnungen am proximalen Ende der spaltbaren Hülse und mehrere Spalter hat.

10. Medizinische Vorrichtung nach einem der Ansprüche 1 bis 7, wobei die spaltbare Hülse eine mit Scharnier angelenkte spaltbare Hülse umfasst, die ein an der expandierbaren Prothese angebrachtes Längsscharnier hat.

11. Medizinische Vorrichtung nach Anspruch 10, wobei die medizinische Vorrichtung mehrere der Betätigungsdrähte, mehrere der Öffnungen am proximalen Ende der mit Scharnier angelenkten spaltbaren Hülse und mehrere Spalter hat.

## Revendications

1. Dispositif médical, comprenant :
un cathéter allongé (4) avec une extrémité proximale et une extrémité distale et une lumière ;
une prothèse expansible (18) disposée autour de ladite extrémité distale dudit cathéter allongé ;
une gaine clivable (10, 110) avec une extrémité proximale et une extrémité distale disposée autour de ladite prothèse expansible ; ladite gaine clivable ayant une ouverture (16) disposée sur ladite extrémité proximale de ladite gaine clivable ;
au moins un fil métallique d'actionnement (20) avec une première portion (22), une deuxième portion (24) et une première extrémité (26) ;
ladite première extrémité étant disposée sur ladite première portion dudit fil métallique d'actionnement ; ladite première portion dudit fil métallique d'actionnement étant retournée autour de ladite extrémité distale dudit cathéter allongé et étant disposée entre ladite prothèse expansible et ladite gaine clivable ; ladite deuxième portion dudit fil métallique d'actionnement étant disposée à l'intérieur de ladite lumière dudit cathéter allongé ;
**caractérisé par** un séparateur (28) attaché à ladite première extrémité dudit au moins un fil métallique d'actionnement ; ledit séparateur comportant un élément de lame (42) ; ledit séparateur étant disposé en partie à l'intérieur de ladite ouverture sur ladite extrémité proximale de ladite gaine clivable de telle sorte que lorsqu'un opérateur, au niveau de ladite extrémité proximale dudit cathéter allongé, tire ledit fil métallique d'actionnement dans la direction proximale, ledit séparateur se déplace dans la direction distale et ledit élément de lame de séparateur clive la gaine clivable.

2. Dispositif médical selon la revendication 1, dans lequel ledit élément de lame de séparateur est en forme de crochet.

3. Dispositif médical selon la revendication 1, dans lequel ledit élément de lame de séparateur est en forme de couteau.

4. Dispositif médical selon la revendication 1, dans lequel un dispositif protecteur couvre ledit séparateur.

5. Dispositif médical selon la revendication 1, dans lequel un cathéter extérieur est disposé autour de ladite gaine clivable ; ledit cathéter extérieur étant configuré de manière à être déplacé dans la direction proximale avant que ladite gaine clivable ne soit clivée.

6. Dispositif médical selon la revendication 1, dans lequel ladite gaine clivable est composée de polytétrafluoréthylène (PTFE) ou de polyéthylène (PE).

7. Dispositif médical selon la revendication 1, dans lequel un affaiblissement longitudinal est disposé sur ladite gaine clivable ; ledit affaiblissement longitudinal s'étendant distalement depuis ladite ouverture sur ladite extrémité proximale de ladite gaine clivable.

8. Dispositif médical selon la revendication 1, dans lequel une ouverture est disposée sur ladite extrémité distale dudit cathéter allongé ; ladite extrémité distale de ladite gaine clivable est attachée à ladite extrémité distale dudit cathéter allongé en position distale par rapport à ladite ouverture sur ladite extrémité distale dudit cathéter allongé ; et ladite première portion dudit fil métallique d'actionnement passe à travers ladite ouverture sur ladite extrémité distale dudit cathéter allongé.

9. Dispositif médical selon la revendication 1, dans lequel ledit dispositif médical présente une pluralité desdits fils métalliques d'actionnement, une pluralité desdites ouvertures sur ladite extrémité proximale de ladite gaine clivable et une pluralité de séparateurs.

10. Dispositif médical selon l'une quelconque des revendications 1 à 7, dans lequel ladite gaine clivable comprend une gaine clivable articulée ayant une charnière longitudinale attachée à ladite prothèse expansible.

11. Dispositif médical selon la revendication 10, dans lequel ledit dispositif médical présente une pluralité desdits fils métalliques d'actionnement, une pluralité desdites ouvertures sur ladite extrémité proximale de ladite gaine clivable articulée et une pluralité de séparateurs.
